# EUROPEAN PATENT APPLICATION

(11) **EP 3 840 223 A1**
(43) Date of publication of application: **23.06.2021**
(21) Application number: 19216549.6
(22) Date of filing: 16.12.2019
(51) Int. Cl.: H03K 3/017

(54) **DUTY CYCLE CORRECTION CIRCUIT AND APPLICATIONS THEREOF**

(71) Applicant: ams International AG, 8645 Jona (CH)
(72) Inventor: Andreou, Charalambos, 8640 Rapperswil (CH)
(74) Representative: Epping - Hermann - Fischer

(57) **Abstract**

A duty cycle correction circuit comprises a buffer stage (120) which outputs a digital output signal (VOUT_RX) having a duty cycle. At least one buffer (125) of the buffer stage (120) is configured to exhibit a controllable tripping threshold. A control loop circuit (130) comprises a sensing circuit (133) including a switched capacitor (410) that is reset to a reference potential and time-integrates the digital output signal (VOUT_RX). A comparator (134) is configured to compare the potential at a terminal of the capacitor with the reference potential. A register stores a correction value determined by the comparator (134) to adjust the tripping threshold of the at least one buffer (125).

## Description

### Technical Field

The present disclosure relates to a duty cycle correction circuit. Specifically, the present disclosure relates to a duty cycle correction circuit that includes a buffer stage and a control loop to generate a digital output signal that has an adjusted duty cycle. The present disclosure also relates to a low voltage differential signalling receiver including a duty cycle correction circuit, a medical imaging apparatus including a low voltage differential signalling receiver and a data processing apparatus including a low voltage digital signalling receiver.

### Background

The duty cycle of a digital signal is indicated by the ratio between the high and low portions of a period of the digital signal. A substantially balanced duty cycle is useful during the processing of a digital signal to achieve a balanced decision window to treat the high and low portions of the signal. During the transmission of a digital signal over a transmission line, the duty cycle may deviate due to mismatches of the transmission line, non-common mode electromagnetic interference and other effects. Furthermore, the digital signal receiver may cause duty cycle deviations due to variations of the p-side and n-side circuit portions and corresponding differences in the rise and fall times of differential signal paths which may be caused by variations of the manufacturing process, of the supply voltage and of the operating temperature as well as non-ideal matching of differential interconnects within the circuit layout. A balanced duty cycle of the digital signal can improve the operation of the circuit. With increased operating frequency, the decision window and the duration of an impulse in a digital signal becomes smaller so that a balanced duty cycle becomes even more necessary.

The digital signal may be a clock signal having a regular pattern of high and low phases of the digital signal. The digital signal may also include data so that the high and low phases of the signal may be irregular in dependence on the transmitted information. Clock signals and data signals may be transmitted over the same transmission line one after the other so that a duty cycle corrected clock signal improves the processing of the transmitted data signal.

There is a need for a duty cycle correction circuit that balances the duty cycle of a received digital signal. The duty cycle correction circuit should perform the duty cycle correction as accurately as possible, substantially independent from process, voltage and temperature impacts and substantially independent of any offsets inherent in the circuit.

It is an object of the present disclosure to provide a duty cycle correction circuit that operates accurately.

It is another object of the present disclosure to provide a duty cycle correction circuit that operates independent from variations of process, supply voltage and operating temperature.

It is yet another object of the present disclosure to provide a duty cycle correction circuit that provides a balanced duty cycle with high correction resolution and achieves the balanced duty cycle quickly.

It is yet another object of the present disclosure to provide a low voltage differential signalling receiver that provides enhanced duty cycle correction.

It is yet another object of the present disclosure to provide a medical imaging apparatus and a data processing apparatus that operate reliably at increased operation speed.

### Summary

One or more of the above-mentioned objects are achieved by a duty cycle correction circuit comprising the features of present claim 1.

According to an embodiment, a duty cycle correction circuit comprises a buffer stage that receives a digital input signal at an input terminal. The digital input signal exhibits a duty cycle. The duty cycle is indicated by the ratio between the binary portions of the digital signal such as the ratio between the high and low phases within a determined duration such as a signal period. The digital signal may be a clock signal. The digital signal may also be a data signal including information to be treated. The buffer stage further comprises an output terminal at which a digital output signal is provided that has a controlled, adjusted duty cycle. Duty cycle control is performed by at least one buffer within the buffer stage that exhibits a tripping threshold of the buffer which is controllable.

A control loop circuit is provided that determines the original duty cycle from the input signal and determines a correction value so that the tripping threshold of the at least one buffer is controlled in such a way that the duty cycle is corrected towards a more balanced duty cycle. Multiple performances of correction operations in the control loop circuit generate an almost balanced, equalized duty cycle at or close to 50%. Other target duty cycle ratios are also possible.

The control loop circuit comprises a sensing circuit that is coupled to the output terminal of the buffer stage and receives the output signal having the modified duty cycle. The sensing circuit includes a switched capacitor which carries a charge or a potential at the end of a sensing cycle that is representative of the duty cycle within the signal sensed by the sensing circuit. The sensing circuit is configured to reset the capacitor to a reference potential and, then, to time-integrate the digital signal into the capacitor. The time integration amplifies the difference between the reference potential to which the capacitor was reset and the amount of duty cycle deviation. The longer the sensing cycle, the larger the amplified difference.

The control loop circuit further comprises a comparator that has an input which is coupled to the capacitor of the sensing circuit. The comparator compares the charge in the capacitor or the potential at one capacitor node with the reference potential to which the capacitor was originally reset. The sign of the comparison indicates whether the duty cycle is too large or too small compared to the target value defined by the reference potential.

The sensing circuit further comprises at least one register which stores a correction value determined by the comparator. The stored correction value is used to control the tripping threshold of the at least one buffer in the buffer stage. With a correction of the tripping threshold of the buffer, the duty cycle of the digital output signal is modified and adjusted to move towards the target value, e.g., balanced or equalized at 50% or close to 50%.

The switched capacitor of the control loop circuit receives the buffered digital input signal, which may be a clock signal, directly. No other active elements are between the sensing capacitor and the to be corrected clock signal so that distortions by process, voltage and temperature (PVT) or offset errors are avoided. The sensing circuit uses a single capacitance for time integration which is reset to the reference voltage at every sensing and correction cycle. This prevents the sensing capacitor from going into saturation. The sensing circuit comprises high side and low side current sources connected to a terminal for supply potential and a terminal for ground potential, respectively, and the current sources are selectively connected to the sensing capacitor in response to the digital output signal of the buffer stage which is the input signal to the control loop and the input signal to the sensing circuit. The capacitor is either charged or discharged through the corresponding current sources in dependence on the high and low phases of the digital input signal to perform the time integration process. Multiple charging and discharging events amplify the duty cycle deviation from the target duty cycle which is set by the reference potential. A large time integration period including a high number of charging and discharging events leads to a high amplification rate and higher accuracy at the cost of longer processing time.

The reference potential may be a common mode potential which is indicated by the potential approximately at the middle of the supply voltage which is half way from both the ground potential and the supply potential. The capacitor is reset to the common mode potential beginning a new sensing cycle and after expiry of a number of previous periods of the output signal from a previous sensing cycle. Resetting the capacitor to the common mode voltage after every sensing cycle and comparing the time integrated capacitor potential with the common mode voltage by the comparator makes the accuracy almost independent of variations of the reference voltage. A simple circuit such as a resistive divider can be used to generate the common mode voltage from the supply voltage. The fact that the sensing starts with a capacitor potential at common mode which may be VDD/2 in the middle of the supply voltage range, allows the comparator to perform with less offset. The effect of comparator offset, which is substantially unavoidable in any circuit mainly due to PVT and mismatch variations, is more critical as it gets closer to ground or VDD potentials and reduced at or around common mode. Feeding the single-ended digital output signal directly to the sensing capacitor, wherein the capacitor is reset to common mode potential, achieves that the potential of the capacitor at the end of a sensing cycle is the common mode potential plus a potential contribution from the duty cycle deviation. This makes the sensing operation very robust. A large number of time integration sensing cycles can be initiated before a correction cycle to achieve a higher correction resolution by amplifying the duty cycle error adequately. The comparator offset becomes insignificant when the amplified duty cycle error is high.

According to embodiments, the sensing circuit may comprise current sources to charge and discharge the sensing capacitor. A first current source may be connected in series with a first switch and to the terminal for supply potential, a second current source may be connected in series with a second switch and to the terminal for ground potential. The capacitor is connected to a node disposed between the first and second current sources and between the first and second switches. Another terminal of the sensing capacitor is connected to the terminal for ground potential. A voltage divider generates the reference potential such as the common mode voltage from the voltage supply and is connected to the capacitor through a third switch which is operated when the sensing capacitor is to be reset to reference/common mode potential. The first and second switches connected serially with the current sources are coupled to the output terminal of the buffer stage and receive the digital output signal. The first and second switches may be coupled to the output terminal of the buffer stage via buffers to receive the buffered digital output signal. The first and second current sources are parts of corresponding current mirror circuits which generate a defined high side and low side current for charging and discharging the sensing capacitor in response to the duty cycle of the output signal.

The comparator may be a chopped clock-driven comparator. The comparator may comprise first and second differential branches, wherein the input terminals of the comparator are alternately coupled to either one of the first and second differential branches in response to a chopping signal. Chopping may be made with every comparison operation. The output signal is retrieved from either one of the first and second branches. A chopped comparator makes a maximum of one consecutive wrong decision in case of an input offset of the comparator. Accordingly, the maximum duty cycle error may be a single step of the equalization process and is not dictated by the comparator input offset. The maximum error can be further minimized by reducing the width to length (W/L) ratio of the transistors of the tripping threshold control of the buffer stage, wherein the W/L ratio of the inverter transistors is a measure for the resolution of the duty cycle control.

The buffer stage includes at least one buffer which is a switchable path connected between the supply potential and ground potential terminals. Furthermore, another switchable path is provided which is connected between the supply and ground potential terminals and the input and output terminals of the switchable path of the at least one buffer. The other switchable path includes first and second switches which are controlled by the stored correction value. Accordingly, the activation of the other switchable path between input and output terminals and one of the supply and ground potential terminals by closing the corresponding switch inserts an asymmetry into the buffer so that the tripping threshold of the combination of switchable path of the buffer and the other switchable path is modified in dependence on the correction value. An asymmetrical tripping threshold of a switchable path changes the duty cycle of the digital signal propagating therethrough. The buffer stage may comprise multiple buffers serially connected in a buffer chain. The input signal is fed into one end of the buffer chain and the digital output signal is provided at another end of the buffer chain. The buffers within the buffer chain have a controllable tripping threshold, wherein the tripping threshold is controlled in response to a correction value determined by the sensing circuit and the comparator, which cause a correction of the duty cycle of the output signal towards a target duty cycle. The target duty cycle is a balanced or almost balanced duty cycle at or close to 50%.

The correction values to control the driving strength of the buffers of the buffer chain may be stored in a shift register, wherein each output of the shift register controls the other switchable paths associated and connected to at least a subset of the buffers. The value stored in one of the shift registers connects or disconnects the input and outputs of the buffers to one of the supply voltage rails through the corresponding switches of the other switchable path so that the driving strength towards the high side or the low side can be increased causing an asymmetry of the corresponding tripping threshold.

According to an embodiment, the buffer stage may comprise a plurality of inverters which are serially connected with each other. A plurality of other inverters is connected through switches to the terminals for supply and ground potential and has input and output terminals that are connected to the input and output terminals of at least a subset of the inverters of the buffer stage. A plurality of registers such as a shift register is provided wherein each one of the registers is associated with one of the other inverters to control the switches therein. Adjusting the tripping threshold of the inverters is achieved in dependence on the correction values stored in the shift register which are determined by the comparator by the repetitive sensing and comparison operation. Several possibilities are conceivable concerning the association of other inverters and buffer inverters. Another inverter can be associated to each one of the buffer inverters. Alternatively, another inverter can be associated to every second one of the buffer inverters. In this case, another inverter can be associated to every odd numbered buffer inverter in the buffer chain or another inverter can be associated to every even numbered buffer inverter. In this case, the correction values stored in the shift register must be matched to the phase sign of the buffer inverters. The possibilities can be combined with each other.

In an exemplary embodiment, the tripping threshold of each one of the buffers is modified by modifying and adjusting the driving strength of the p-MOS and n-MOS sides of the buffer inverters in accordance with the decision taken by the comparator based on the results of the sensing mechanism. Modifying the driving strength has only little or almost no impact on the propagation delay of the signal through the buffer chain. The resolution can be adjusted in that the size of the transistors of the other inverters is adapted by setting the W/L (width/length) ratio of said transistors which may be the ratio of width and length of the gate area. A small W/L ratio provides a fine resolution of duty cycle correction steps.

Concerning the power consumption of the duty cycle correction circuit, it is to be noted that the control loop will operate only for a few microseconds, for example, approximately 1 to 3 µs for the correction of a clock signal of 0.5 GHz. The operation time depends on the actual required resolution. For a resolution of +/- 2%, the required time may be about 1 µs.

The regulation loop goes to sleep after finalizing the duty cycle correction when a steady state condition is met and then waiting for a possible future enable signal. During sleep mode, the only circuitry from the correction circuit that is active are the registers such as the shift register and the buffer chain including the other switchable paths/inverters for tripping threshold control. The sensing circuit and the comparator may be switched off during steady state as well as the frequency division and synchronizing circuit and the timing and sequencing circuit. The overall power consumption is therefore substantially insignificant. The switch off of the corresponding circuit blocks in the control loop may be performed after expiry of a certain amount of time which may be determined by simulation to make sure that the duty cycle is sufficiently balanced. The control loop circuits may be switched on after expiry of another predetermined amount of time to re-correct the duty cycle. Alternatively, other strategies to determine steady state may be used which may include that the duty cycle deviation is below a certain level. The circuit can be switched on when an environmental condition changes such as voltage and/or temperature. A signal representative of ambient or chip temperature may already be available on system level for other reasons.

The control loop circuit is also applicable for double data rate (DDR) transmission, wherein a clock signal can be transmitted for a limited time for only, for example, 1 to 3 µs during a startup phase so that the balancing code may be saved in the registers and the data transmission is continued thereafter.

The duty cycle correction circuit may be used in a receiver circuit such as a low voltage differential signalling receiver (LVDS-RX). A LVDS-RX comprises an input terminal for a differential input signal such as a clock signal or a data signal or an interleaved transmission of clock and data signals. An amplification stage receives the differential input signal and generates an amplified single-ended output signal which is forwarded to the duty cycle correction circuit to generate a single-ended duty cycle corrected output signal having a balanced, equalized duty cycle. The LVDS-RX may be located at the input section on an integrated circuit to receive the signals from the printed circuit board with a low amplitude via a transmission line. The duty cycle correction circuit recovers the signal with equalized duty cycle and rail-to-rail amplitude, wherein the duty cycle correction circuit according to the principles of the present disclosure is substantially independent from PVT variations.

An exemplary field of application of a LVDS-RX is in medical imaging that uses radiation such as x-ray radiation, for example, in a computer tomography apparatus. The radiation in a medical imaging apparatus such as a CT apparatus generates x-ray radiation for the treatment of a living organism such as a human being or an animal to generate an image of a portion of the living organism. In medical imaging type apparatuses there is always a need to increase operating speed since higher operating speed allows the real-time processing of larger images with higher resolution. The LVDS-RX according to the principles of the present disclosure allows to correct the duty cycle to a balanced level so that the operating speed can be increased due to the increased accuracy of the corrected signal. While radiation events may disturb the duty cycle of a transmitted signal, for example, when transmitted from the image sensor to a post processing circuit, the duty cycle correction circuit according to the principles of the present disclosure reestablishes a balanced duty cycle when distorted by a radiation event. The circuit operates fast and robust in a radiation-contaminated environment.

A LVDS-RX including a duty cycle correction circuit according to the principles of the present disclosure may be also used in the field of data processing where a processor is to be connected to a display device to display information treated and generated by the processor. Such display devices may have a serial interface that has an elevated operating speed to transmit all the required information to operate the display. The interface may comply with the specifications of the Mobile Industry Processor Interface (MIPI) Alliance for Display Serial Interface (DSI). The duty cycle correction circuit and the corresponding LVDS-RX provides a robust, PVT-resistant high speed differential signalling point-to-point serial bus. A MIPI DSI transmission comprises a speed clock lane and one or more data lanes to transmit information to be displayed from a processor to the serial interface of a display device such as a display screen.

It is to be understood that both the foregoing general description and the following detailed description are merely exemplary, and are intended to provide an overview or framework to understand the nature and character of the claims. The accompanying drawings are included to provide a further understanding and are incorporated in, and constitute a part of, this description. The drawings illustrate one or more embodiments, and together with the description serve to explain principles and operation of the various embodiments.

The same elements in different figures of the drawings are denoted by the same reference signs.

### Brief Description of the Drawings

In the drawings:
Figure 1 shows a principle block diagram of a LVDS receiver including a duty cycle correction circuit according to the principles of the present disclosure;
Figure 2 shows a detailed circuit diagram of the frequency division and synchronizing block of the circuit of Figure 1;
Figure 3 shows a detailed circuit diagram of the timing and sequencing block of Figure 1;
Figure 4 shows a detailed circuit diagram of the sensing circuit of Figure 1;
Figure 5 shows a common mode voltage generator to be used in the sensing circuit of Figure 4;
Figure 6 shows a detailed circuit diagram of the chopped comparator of Figure 1;
Figure 7 shows a detailed circuit diagram of the storage circuit and the tripping threshold control of the buffer chain of Figure 1;
Figure 8 shows a waveform diagram of signals used in the circuits of Figures 1 to 7;
Figure 9 shows a simulation of signals used the circuits of Figures 1 through 7;
Figure 10 shows an example of the digital output signal at the beginning of a duty cycle correction operation;
Figure 11 shows an example of the digital output signal after a duty cycle correction;
Figure 12 shows a CT apparatus including a LVDS receiver; and
Figure 13 shows a data processing apparatus including a LVDS receiver.

### Detailed Description of Embodiments

The present disclosure will now be described more fully hereinafter with reference to the accompanying drawings showing embodiments of the disclosure. The disclosure may, however, be embodied in many different forms and should not be construed as limited to the embodiments set forth herein. Rather, these embodiments are provided so that the disclosure will fully convey the scope of the disclosure to those skilled in the art. The drawings are not necessarily drawn to scale but are configured to clearly illustrate the disclosure.

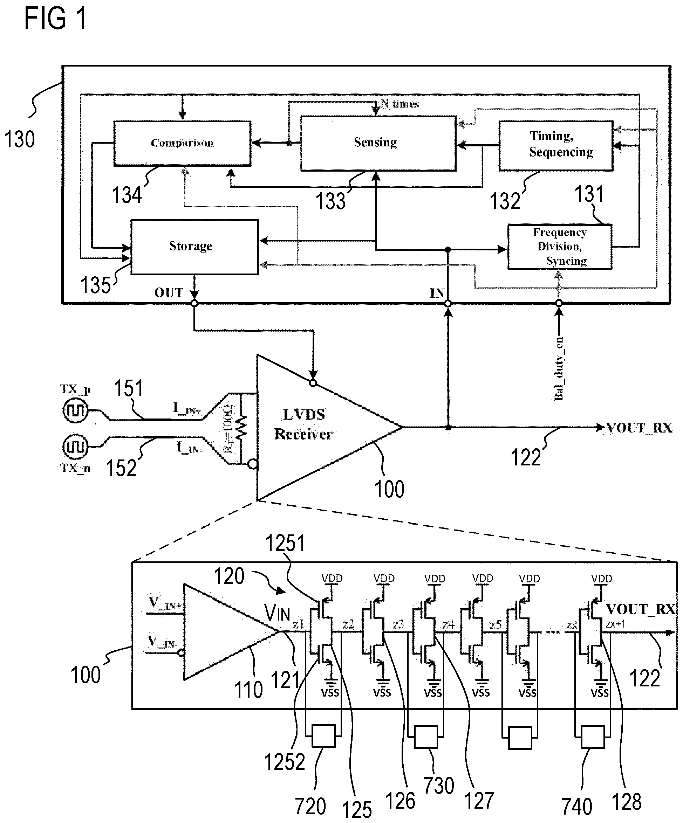

Figure 1 shows a block diagram of a LVDS receiver including a duty cycle correction circuit according to the principles of the present disclosure. The duty cycle correction circuit may also be used in other circuits where duty cycle correction is required. The LVDS receiver 100 may be used as an input port to an integrated circuit chip receiving a differential signal from a transmission line disposed on a printed circuit board. The input terminals of receiver 100 are connected to corresponding wires 151, 152 that carry the positive and negative portions of the differential signal to transmit clock and data from signal sources TX_p and TX_n. The signal is transmitted over the transmission wire line as a current signal I_IN+, I_IN-. The transmission wire line is terminated by a resistance of 100 Ω so that the receiver obtains differential input signals V_IN+, V_IN-. Receiver 100 includes a receiver core 110 connected to the receiver inputs to amplify the low voltage differential input signal to a single-ended full swing signal VIN. A buffer stage 120 including a serial connection or chain of buffers 125, 126, 127, 128 is connected downstream the receiver core 110 to generate an output signal VOUT_RX at output terminal 122 of LVDS receiver 100. The receiver core 110 performs amplification of the differential signal in multiple amplification stages, transforming of the differential signal to a single-ended signal, electrostatic discharge protection and failsafe functions which generate a defined signal when the receiver input is floating. The buffer stage 120 includes CMOS inverter buffers such as buffer 125 comprising p- and n-MOS-transistors 1251, 1252 connected between the terminals for supply potential VDD and ground potential VSS, wherein the output of one inverter buffer such as 125 is connected to the input of the downstream connected next buffer such as 126 within the chain. Although only a subset of buffers is shown in Figure 1 for examplary reasons, a buffer stage may contain several tens of buffers such as 30 or 40. In principle, even more buffers in a chain may be used as demanded by the application considering power consumption constraints.

The duty cycle of the received input signal VIN is subject to duty cycle deviation for multiple reasons. For example, the transmission line 151, 152 may have mismatches or may be subject to electromagnetic interference or the transmitters TX_P, TX_N may already generate a signal of non-balanced duty cycle. Furthermore, the LVDS receiver may introduce a duty cycle deviation, for example, by different rise and fall times of its p- and n-MOS circuits which may be due to process, voltage and temperature (PVT) impacts, non-ideal matching of differential interconnects within the layout and others.

The duty cycle correction circuit is provided to generate an output signal VOUT_RX having a corrected duty cycle from the input signal VIN. The duty cycle correction circuit includes a control loop which repetitively senses the duty cycle deviation of the output signal VOUT_RX and corrects the deviation so as to achieve a balanced, equalized duty cycle which is 50% or close to 50% after a number of repetitive correction cycles. The control loop circuit comprises a sensing circuit 133 that receives the output signal VOUT_RX from output terminal 122. A comparison circuit 134 compares the sensed duty cycle level with a reference level that indicates the wanted target duty cycle level. A storage circuit 135 provides correction values which are obtained from the comparison stage 134. The stored correction values control the buffer stage 120 so that the duty cycle of the output signal VOUT_RX is adjusted and the control loop operation including sensing, comparing and storing recommences and the loop operation is repeated until a steady state condition is achieved.

The correction values from storage block 135 control the tripping thresholds of the individual buffers 125, ..., 128 within the buffer chain 120 so that rising and falling edges can be selectively accelerated or decelerated and the duty cycle as the ratio of low and high portions of a signal period is modified and adjusted. The differential input signal to the LVDS receiver V_IN+, V_IN- as well as the input signal to the buffer chain VIN can be a clock signal having high to low and low to high transitions at the operating frequency. The input signal may also be a data signal in which transitions are governed by the transmitted information. The duty cycle correction is performed on the clock signal and the correction values can be stored in storage 135 so that the data signals can be received thereafter using the tripping threshold setting of the buffer stage determined beforehand. The control mechanism in the buffer chain employs adjusting of the tripping thresholds which correct one of the root causes of duty cycle deviations caused by the asymmetry of p- and n-side components and transistors. The control concept of the present disclosure avoids the addition of additional delays which could reduce the resolution since there is a certain design limit that dictates the minimum possible delay.

Adjustment of the tripping thresholds of buffers 125, ..., 128 is achieved by switchable paths 720, 730, 740 connected to the input and output terminals of the buffers 125, ..., 128 of buffer stage 120 such as terminals z1, z2, z3, z4, z5, ... zx, zx + 1. The switching states of the switchable paths 720, 730, 740 are controlled by the storage circuit 135. The details of the switchable paths 720, 730, 740 are explained in more detail hereinbelow. The switchable paths can be connected in parallel to the input and output terminals of every second buffer such as the first, the third, the fifth etc. buffer in the buffer chain as depicted in Figure 1. Alternatively, the switchable paths can be connected in parallel to the inputs and outputs of the second, the fourth, the sixth etc. buffer in the buffer chain. It is also possible to connect a switchable path in parallel the inputs and outputs of every buffer in the buffer chain.

When a steady state condition is achieved, for example, after performance of several correction cycles or when it is determined that the duty cycle deviation is below a resolution criterion, most portions of the control loop circuit 130 can be switched off to save power. For example, the sensing and comparison circuits 133, 134 as well as the frequency division and synchronizing circuit 131 and the timing and sequencing circuit 132 can be switched off. This considerably reduces the amount of power consumed. The storage circuit 135 including storage registers is the only circuit that remains active and stores the correction values to control the tripping threshold of the buffers in the buffer stage 120. The power consumption of these registers is negligible, as they do not switch during steady state. The control loop circuit can wake up after expiry of a predetermined amount of time determined by a timer or in response to an external event such as a variation in supply voltage or a change of temperature indicated by a temperature sensor available on system level. Such an event will activate the signal Bal_duty_en to reactivate the circuit blocks of the control loop.

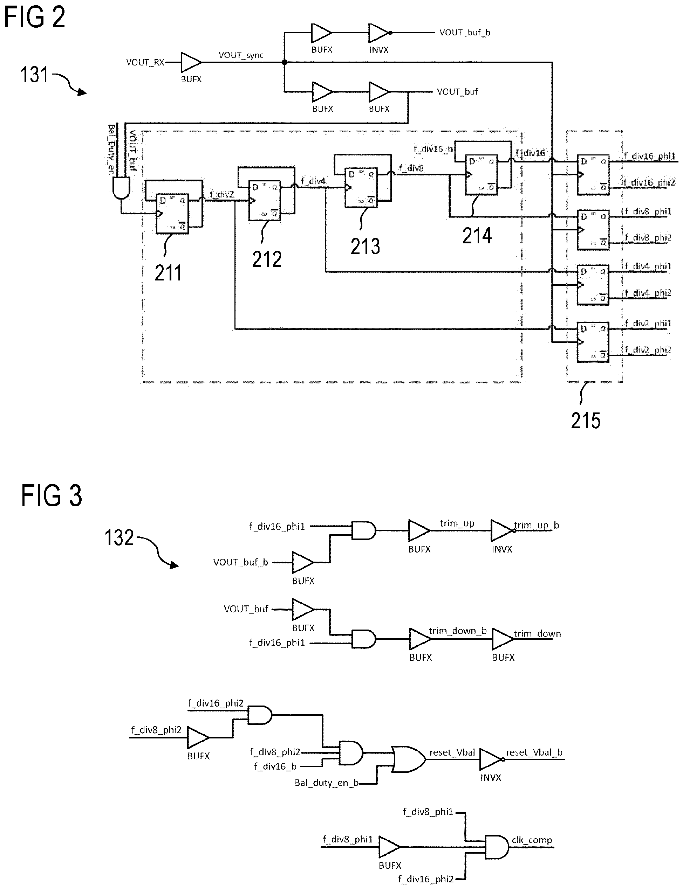

In the following, several blocks from the high level representation shown in Figure 1 are explained thereafter in Figures 2 through 7 on the basis of detailed circuit representations.

Figure 2 shows a detailed circuit diagram of the frequency division and synchronizing block 131. The circuit receives the output signal VOUT_RX of the buffer stage 120 at terminal 122 which is the input signal to the regulation loop. A buffered version VOUT_buf of this signal is frequency-divided by four flip-flops 211, 212, 213, 214 to generate the clock frequency divided by two, the clock frequency divided by four, the clock frequency divided by eight and the clock frequency divided by sixteen. Other clock division circuits are also possible, such as non-overlapping clock generators. Several flip-flops 215 are used to synchronize the divided clock signals with the output clock signal and generate synchronized versions of the divided clock signals f_div2_phi1, ..., f_div16_phi1. Complementary divided synchronized clock signals f_div2_phi2, ..., f_div16_phi2 are also provided by the flip-flops 215.

Figure 3 shows a circuit representation of the timing and sequencing block 132. Timing and sequencing block 132 generates control signals that are used to control operation of the other blocks 133, 134, 135. The control signals are generated from the divided clock signals and the buffered output clock signal. An enable signal Bal_duty_en_b enables the overall operation of the control loop circuit. This enable signal can be activated, for example, in response to the switching on of the system, a change of an environmental condition, the expiry of a timer and others.

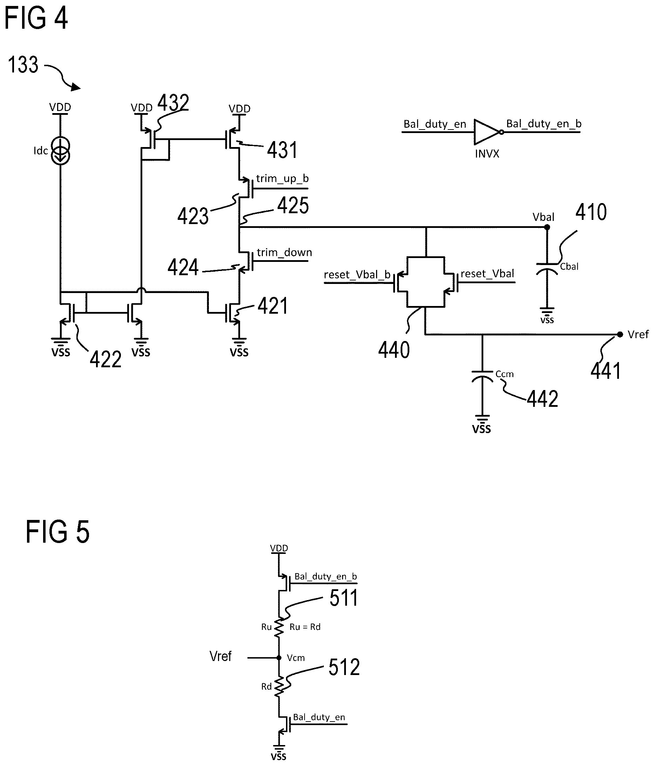

Figure 4 shows a detailed circuit diagram of the sensing circuit 133. The sensing circuit 133 comprises a switched capacitor 410 which is used to time-integrate the digital output signal VOUT_RX so that capacitor 410 carries a charge that is representative of the duty cycle deviation. The capacitor 410 is connected to respective current sources 431, 421 which are connected to the terminal for supply potential VDD and the terminal for ground potential VSS, respectively. A corresponding switch 423, 424 is connected between the current sources 431, 421 and a node 425 which is connected to the capacitor 410. Capacitor 410 is connected between node 425 and the terminal for ground potential VSS. The switches 423, 424 are controlled by signals trim_up_b and trim_down from timing and sequencing block 132 which are basically derived from the buffered output signal VOUT_RX. Accordingly, switched capacitor 410 is selectively connected to current sources 431, 421 through switches 423, 424 in response to the output signal so that the potential Vbal at capacitor 410 is representative of the deviation of the duty cycle from a reference level which may be a balanced, equalized duty cycle. The current sources 431, 421 are portions of current mirrors which are fed by a corresponding current Idc which is mirrored through diode-connected transistor 422 of the input branch of a first current mirror into current source 421 and through diode connected transistor 432 of the input branch of a second current mirror controlling the current through current source 431.

Capacitor 410 is reset to a reference potential VREF at terminal 441 through a switch 440 at the beginning of a sensing cycle. Capacitor 442 smoothes the signal VREF and provides sufficient charge to quickly drive the potential Vbal at capacitor 410. The reference potential VREF may be any reference potential in between the voltage supply rails VDD, VSS. In an embodiment, the reference potential VREF is the common mode voltage Vcm which is basically the middle voltage between the voltage supply rails VDD, VSS. The common mode voltage Vcm can be generated by a resistive divider 511, 512 as shown in Figure 5. Resetting the switched capacitor 410 to the common mode voltage at the beginning of a sensing cycle allows a fast operation so that after a certain number of charging and discharging steps of capacitor 410 in response to the output signal VOUT_RX the capacitor 410 and the potential Vbal carry the information of the duty cycle deviation. The difference between the potential Vbal at capacitor 410 and the resetting common mode voltage Vcm contains the information of the duty cycle deviation from the balanced level. The higher the number of integration cycles, the higher the amplification of the deviation of the duty cycle. The resetting of the circuit 410 after a number of time integrating steps prevents the sensing circuit 153 from going into saturation.

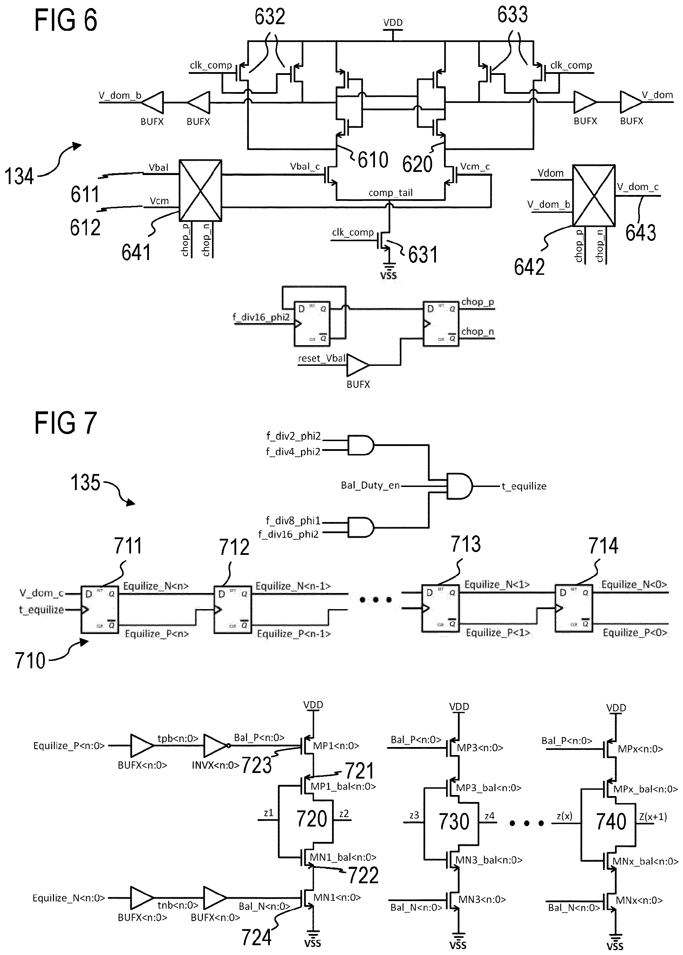

Figure 6 shows a detailed circuit diagram of the comparator 134. The comparator comprises first and second differential branches 610, 620 which receive the reference signal which is the common mode signal Vcm in the present example and the potential Vbal at the switched capacitor 410 representative of the deviation of the duty cycle. The comparator is clock controlled by signal clk_comp so that the low side current source 631 can be switched off. Also the high side paths can be switched off through transistor 632, 633. The power consumption is reduced as comparator 134 consumes power only when a comparison operation is performed and is switched off during an idle time period. Furthermore, the comparator 134 is operated in chopped mode so that the input signals Vbal and Vcm can be exchanged between the branches 610, 620 with every decision through a chopping circuit 641.

Correspondingly, the output signal v_dom_c at output terminal 643 is exchanged between branches 610, 620 through chopping circuit 642. As any comparator has an inherent offset which may be caused by the mismatch between the branches 610, 620, the maximum offset error is incurred only once in an initial comparison and the chopping operation avoids further offset errors since the function of the branches is exchanged. The comparator determines whether the duty cycle error represented by the capacitor potential Vbal is above or below the common mode potential Vcm and generates a corresponding output signal V_dom_c to correct the tripping threshold of one or more of the buffers of the buffer chain 120.

Figure 7 shows a detailed diagram of the storage circuit 135 and the switchable paths 720, 730, 740 for tripping threshold control of the buffer chain 120. The storage circuit 135 is realized by a shift register 710 of which four register stages 711, 712, 713, 714 are shown connected serially. After finalizing an integration and comparison operation in sensing and comparator circuits 133, 134, the output signal V_dom_c from comparator 134 representing a correction value is fed into the shift register 710 and forwarded through the shift register with every next feeding operation. One register stage of the shift register is associated with one instance <n> of every switchable path. Every switchable path includes <n> instances each controlled by a register. For example, register 711 is associated with transistors <n> of switchable paths 720, 730, ..., 740 so that signal Equalize_P<n> controls p-MOS transistors MP1<n>, MP3<n>, ..., MPx<n> and signal Equalize_N<n> controls n-MOS transistors MN1<n>, MN3<n>, ..., MNx<n>. Register 712 is associated with transistors <n-1> of switchable paths 720, 730, ..., 740, register 713 with transistors <1> of switchable paths 720, 730, ..., 740 etc.

When signal Equalize_N<n> is H (High), transistor MN1<n> is conducting so that transistor MN1_bal<n> is enabled adding additional driving strength of n-MOS transistor MN1_bal<n> to the n-MOS-transistor 1252 of buffer 125 increasing the pull down drive capability of transistor 1252. In this case, transistors 1252 and MN1_bal<n> are connected in parallel to each other. At the same time, signal Equalize_P<n> is L (Low) so that the gate of transistor MP1<n> is driven H by BUFX<n> and INVX<n> shutting off transistor MP1<n> not adding any drive capability to the p-MOS side transistor 1251 of inverter 125. When signal Equalize_N<n> is L, transistor MN1<n> is shut off. At the same time, signal Equalize_P<n> is H so that the gate of transistor MP1<n> is driven L by BUFX<n> and INVX<n> enabling transistor MP1<n> adding the additional driving strength of p-MOS transistor MP1_bal<n> to the p-MOS-transistor 1251 of buffer 125 increasing the pull up drive capability of transistor 1251. In this case, transistors 1251 and MP1_bal<n> are connected in parallel to each other. Transistors MN1_bal<n> and MP1_bal<n> are connected as an inverter that has input and output terminals that are connected to terminals z1, z2 of one of the buffers of the buffer stage so that they are controlled by the signal of which the duty cycle is to be corrected. Transistors MN1<n> and MP1<n> are connected between said inverter and the terminals for supply and ground potentials to enable said inverter in dependence on the correction signal Equalize_N<n> and Equalize_P<n>. In the same way, transistors MN3_bal<n> and MP3_bal<n> are connected as an inverter that has input and output terminals that are connected to terminals z3, z4 of another one of the buffers of the buffer stage.

Transistors MN3<n> and MP3<n> are connected between said inverter and the terminals for supply and ground potentials to enable said inverter in dependence on the correction signal Equalize_N<n> and Equalize_P<n>. This applies to every instance <n>.

When transistor MP1<n> of switchable path 720 is enabled, at the same time transistor MP3<n> of switchable path 730 and transistor MPx<n> of switchable path 740 are enabled. In the same way, when transistor MN1<n> of switchable 720 is enabled, at the same time transistor MN3<n> of switchable path 730 and transistor MNx<n> of switchable path 740 are enabled. In summary, each of the storage elements 711, 712, ..., 713, 714 controls one instance <n> on every switchable path 720, 730, ..., 740. Each instance <n> comprises one p-MOS transistor and one n-MOS transistor in every switchable path 720, 730, ..., 740 such as p-MOS transistors MP1<n>, MP3<n>, ..., MPx<n> and n-MOS transistors MN1<n>, MN3<n>, ..., MNx<n>.

Figure 7 depicts the version shown in Figure 1, when the switchable paths are connected in parallel to the inputs/outputs of the odd numbered buffers 125, 127, 128. In an alternative version, when the switchable paths are connected in parallel to the inputs/outputs of the even numbered buffers (not shown), the correction signals Equalize_N<n> and Equalize_P<n> must be flipped at switchable path 720 as well as the corresponding signals at switchable paths 730, 740. The indices shown in Figure 7 may comply with the following rules:
n = 1 to N,
x = 1 to X, when x is odd numbered, or
x = 2 to X, when x is even numbered, wherein
Equilize_P and Equalize N are to be flipped.

The width to length ratio (W/L) of the transistors MP1_bal<n:0> and MN1_bal<n:0> represents the driving strengths of said transistors so that the W/L ratio determines the resolution and the step size of the control loop operation and thus determines the dynamic range of the regulation.

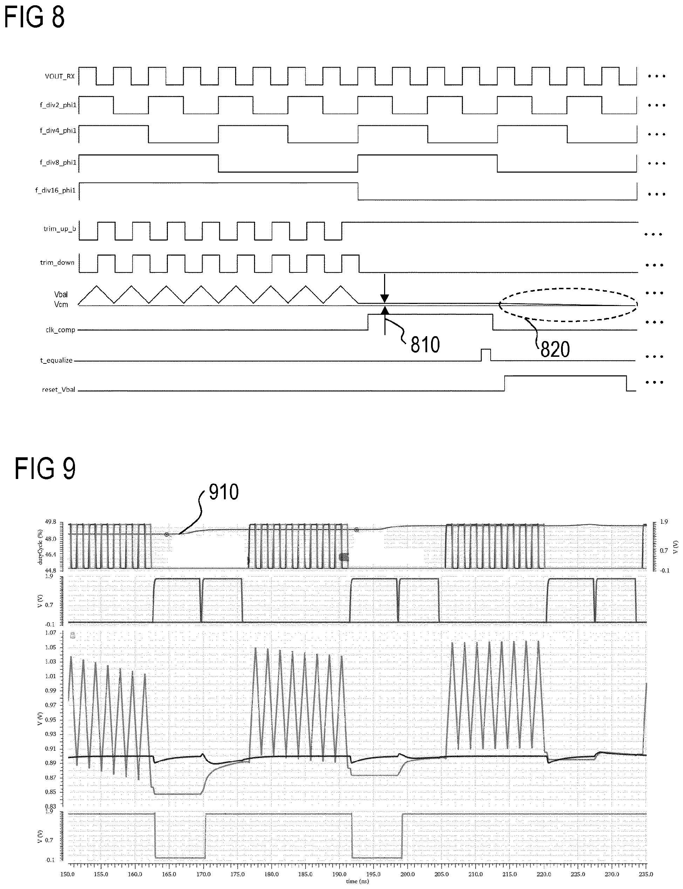

Figure 8 shows a waveform diagram of signals from the circuits shown in Figures 1 to 7. The output signal VOUT_RX is a clock signal having transitions with every clock cycle. The divided clock signals f_div2_phi1, ..., f_div16_phi1 are obtained from the frequency division and synchronizing block 131 and provide divisions of the input clock signal. The signals trim_up_b and trim_down are generated in timing and sequencing block 132 to cause the time-integration of the clock signal by charging and discharging the switched capacitor 410. Charging and discharging is shown with signal Vbal having ramping and falling portions in response to the operation of current sources 431, 421, resp. The resulting deviation 810 between signals Vbal and common mode voltage Vcm is representative of the deviation of the duty cycle from the ideal equalized level represented by common mode reference potential Vcm. The sign of the deviation is determined by comparator 134 in response to signal clk_comp. In response to signal reset_Vbal, the potential Vbal at switched capacitor 410 is returned to the common mode voltage Vcm as shown at 820. Figure 8 depicts a number of eight charging and discharging operations as an example. In general, more charging and discharging operations can be performed achieving a larger amplification of the duty cycle deviation, however, at the expense of a longer processing time. Only one decision cycle including one sensing cycle and one comparing operation is depicted and multiple cycles can be performed consecutively as indicated with the dots at the right end of the waveform diagram until the output signal VOUT_RX achieves a balanced duty cycle. The balanced duty cycle may be 50% or close to 50% due to the finite regulation resolution.

Figure 9 shows a waveform diagram according to a simulation for three measuring and decision cycles. The duty cycle represented by curve 910 is initially around 48.6% and increases close to 49.4% after the third cycle.

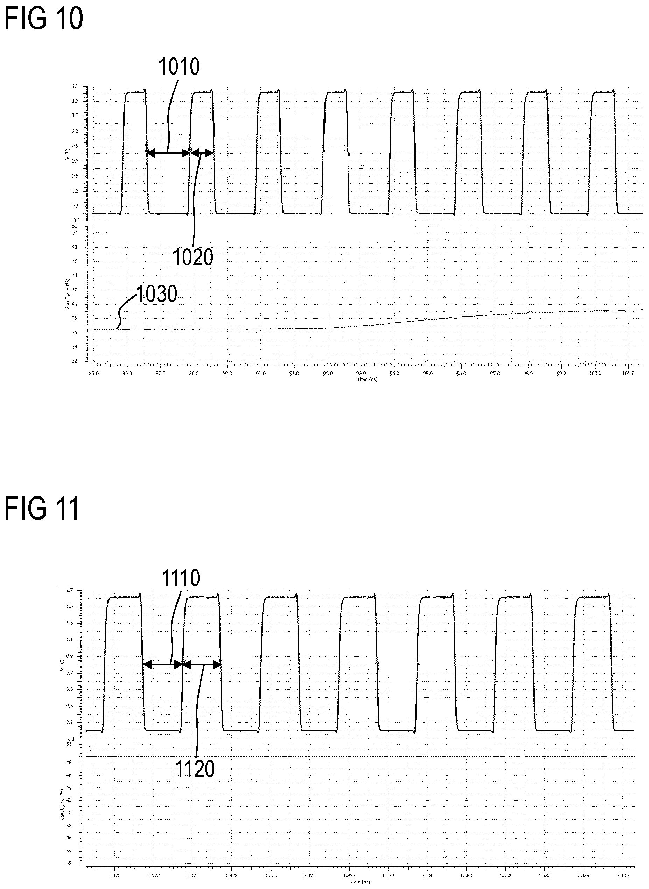

Turning now to Figure 10, the clock signal VOUT_RX at the beginning of a duty cycle correction process is shown. The clock signal exhibits a highly unbalanced duty cycle in that the low clock cycle period 1010 and the high clock cycle period 1020 have a substantially different duration leading to a duty cycle of 36.5% in region 1030 of the curve representing the duty cycle.

Figure 11 shows the situation at the end of a duty cycle correction process. The low period 1110 and the high period 1120 have substantially the same duration so that the duty cycle is at 49%. Although the ideal target duty cycle is at 50%, the steady state may slightly deviate therefrom due to the resolution of the control loop.

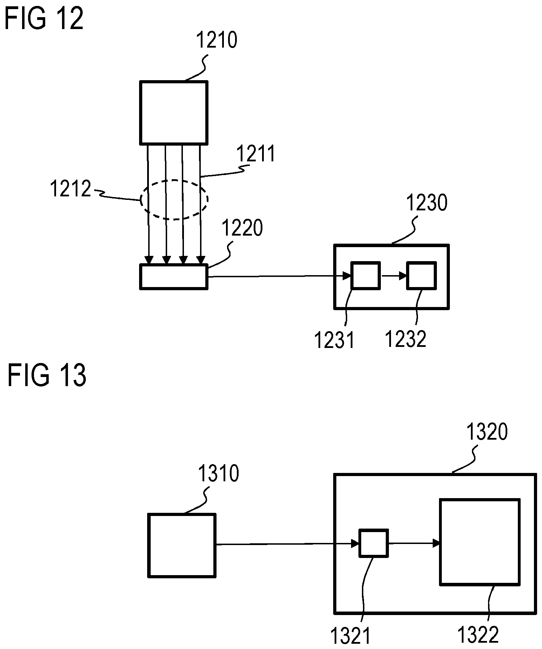

Figure 12 shows the application of a LVDS receiver according to the principles of the present disclosure of Figure 1 in a computer tomography (CT) apparatus. X-ray radiation 1211 is generated by an x-ray source 1210 to investigate a human being or an animal that is positioned in area 1212. The x-ray image sensor 1220 receives the x-ray radiation modified by the living organism. The image sensor 1220 may include x-ray photodiodes, analog-to-digital converters and a digital serial interface to transmit the digital image information to a post-processing device 1230. The sensor 1220 covers a relatively large area and the generated and to be transmitted amount of data is relatively high and at the edge of the available technology since there is always a need to increase the size and resolution of the image sensor to improve medical analysis. The environment is contaminated by the x-ray radiation and, although the circuits may be protected from x-ray radiation, the protection may not be perfect so that there is a risk of radiation reaching the transmission lines and the electronic devices. LVDS receiver 1231 receives the serial data from image sensor 1220 and forwards the data to a post processor 1232. It is important to provide duty cycle correction so that the sensing window for the data received in post processor 1230 works at the optimum. Furthermore, duty cycle distortion may occur due to the x-ray environment. The duty cycle correction control loop according to the principles of the present disclosure and explained in connection with the foregoing figures 1 to 11 allows to detect a duty cycle distortion situation due to an x-ray interference event, enable the duty cycle correction control loop and equalize the duty cycle again.

Figure 13 shows a data processing apparatus such as a computer, a mobile computing device or a smartphone. Processor 1310 generates information to be displayed on a display screen 1320. The information may be received serially at a LVDS receiver 1321 according to the principles of the present disclosure. The LVDS-RX 1321 may comply with an industry standard of serial data transmission such as the Mobile Industry Processor Interface (MIPI) Alliance Display Serial Interface (DSI).

The duty cycle control loop according to the principles of the present disclosure enables a high speed serial communication from processor 1310 to display screen 1320 which is very robust against PVT deviations. The serial clock and data stream received at LVDS-RX 1321 is forwarded to display device 1322 to visualize the transmitted information for optical reception by a user.

It will be apparent to those skilled in the art that various modifications and variations can be made without departing from the spirit or scope of the disclosure as laid down in the appended claims. Since modifications, combinations, subcombinations and variations of the disclosed embodiments incorporating the spirit and substance of the disclosure may occur to the persons skilled in the art, the disclosure should be construed to include everything within the scope of the appended claims.

## Claims

1. A duty cycle correction circuit, comprising:
- a buffer stage (120), comprising:
-- an input terminal (121) for a digital input signal (VIN) having a duty cycle;
-- an output terminal (122) for a digital output signal (VOUT_RX) having a modified duty cycle; and
-- at least one buffer (125), the buffer configured to exhibit a controllable tripping threshold;
- a control loop circuit (130), comprising:
-- a sensing circuit (133) coupled to the output terminal (122) of the buffer stage, comprising a switched capacitor (410) and configured to reset the capacitor to a reference potential (Vref, Vcm) and to time-integrate the digital output signal (VOUT_RX) in the capacitor (410); and
-- a comparator (134) coupled to the capacitor (410) and configured to compare the potential at a terminal of the capacitor (Vbal) with the reference potential (Vref, Vcm); and
-- a register (711, ..., 714) to store a correction value (Equalize_N, Equalize_P) determined by the comparator (134), wherein the tripping threshold of the at least one buffer (125) is controlled in dependence on the correction value.

2. The duty cycle correction circuit according to claim 1, wherein the sensing circuit (133) further comprises a current source (431) connected to a terminal for a supply potential (VDD) and a current source (421) connected to a terminal for ground potential (VSS), the sensing circuit configured to connect the current sources (431, 421) selectively to the capacitor (410) in response to the digital output signal (VOUT_RX, trim_up_b, trim_down) at the output terminal (122) of the buffer stage.

3. The duty cycle correction circuit according to claim 1 or 2, wherein the reference potential is a common mode potential (Vcm) and the sensing circuit is configured to reset the capacitor (410) to the common mode potential (Vcm) after a number of consecutive periods of the output signal.

4. The duty cycle correction circuit according to claim 1, wherein the sensing circuit (133) further comprises a first current source (431) connected to a terminal for a supply potential (VDD) and a first switch (423), a second current source (421) connected to a terminal for ground potential (VSS) and a second switch (424), wherein the capacitor (410) is connected to a node (425) disposed between the first and second current sources (431, 421) and to the terminal for ground potential (VSS), a voltage divider (511, 512) connected to the terminal for a supply potential (VDD) and to the terminal for ground potential (VSS) and connected to the capacitor (410) through a third switch (440), the first and second switches (424, 423) coupled to the output terminal of the buffer stage and the third switch (440) controlled in response to expiry of a number of clock cycles of the digital output signal at the output terminal of the buffer stage.

5. The duty cycle correction circuit according to claim 4, wherein the first and second current sources (431, 421) are each included in an output path of a corresponding current mirror (431, 432; 421, 422).

6. The duty cycle correction circuit according to any of claims 1 to 5,
wherein the sensing circuit (133) is configured to time-integrate the digital output signal in the capacitor (410) during a plurality of cycles of the digital output signal (VOUT_RX) after a resetting (reset_vbal) of the capacitor (410) to the reference potential (Vref, Vcm).

7. The duty cycle correction circuit according to any of claims 1 to 6, wherein the comparator (134) comprises a first differential branch (610) and a second differential branch (620), a first input terminal (611) coupled to the capacitor (410) and a second input terminal (612) coupled to a terminal for the reference potential (Vref, Vcm) and an output terminal (643), wherein the comparator (134) is configured to perform chopping, wherein the first and second input terminals (611, 612) are alternately coupled to the first and second differential branches (610, 620) and the output terminal (643) is alternately coupled to one of the first and second differential branches (610, 620).

8. The duty cycle correction circuit according to any of claims 1 to 7, wherein the at least one buffer (125) comprises a switchable path (1251, 1252) connected between a terminal for a supply potential (VDD) and a terminal for ground potential (VSS) further comprising another switchable path (720) connected to the terminal for supply potential (VDD) through a first switch (723) and to the terminal for ground potential (VSS) through a second switch (724), wherein input and output terminals (z1, z2) of the switchable path (125) and the other switchable path (720) are connected to each other and the first and second switches (723, 724) of the other switchable path (720) are controlled by the correction value (Equalize_N, Equalize_P) stored in the register (711).

9. The duty cycle correction circuit according to any of claims 1 to 8, wherein the buffer stage (120) comprises a chain of buffers (125, 126, 127, 128) serially connected with each other, wherein the input terminal (121) for a digital input signal (VIN) is connected to one end of the chain of buffers (120) and the output terminal for a digital output signal (VOUT_RX) is connnected to another end of the chain of buffers (120), wherein the buffer stage further comprises a plurality of other switchable paths (720, 730, 740), wherein each one of the plurality of other switchable paths is associated with a subset of the buffers of the chain of buffers, wherein input and output terminals (z1, z2; z3, z4; z(x), z(x + 1)) of the other switchable paths and the associated one of the buffers are connected with each other.

10. The duty cycle correction circuit according to claim 9, further comprising a shift register (710), wherein each register (711, 712, 713, 714) of the shift register is connected to at least one or more of the other switchable paths (720, 730, 740), wherein each one of the other switchable paths is configured to be one of connected and disconnected to a terminal for supply potential (VDD) and configured to be one of connected and disconnected to a terminal for ground potential (VSS) in response to the operation of the control loop circuit (130).

11. The duty cycle correction circuit according to any of claims 1 to 7, wherein the buffer stage (120) comprises:
- a plurality of inverters (125, 126, 127, 128) serially connected with each other and
- a plurality of other inverters (721, 722) connected through switches (723, 724) to terminals for supply and ground potentials (VDD, VSS) and having input and output terminals (z1, z2) connected to input and output terminals of at least a subset of the inverters (125, 127, 128);
further comprising:
- a plurality of registers (711, 712, 713, 714), wherein each one of the registers is associated with at least one or more of the other inverters (721, 722) configured to control the switches (723, 724) connected to the associated one of the other inverters (721, 722) to adjust a tripping threshold of the associated one of the other inverters (721, 722) in dependence on correction values (Equalize_N, Equalize_P) determined by the comparator (134) .

12. The duty cycle correction circuit according to any of claims 1 to 11, wherein a portion of the control loop circuit including the sensing circuit (133) and the comparator (134) is switched off, when a steady state condition is achieved.

13. A low voltage differential signalling receiver (100), comprising:
- an input terminal for a differential digital input signal (V_IN+, V_IN-);
- an amplification stage (110) having an output terminal for a single ended amplified signal (VIN);
- the duty cycle correction circuit of any of claims 1 to 12, wherein the input terminal (121) of the duty cycle correction circuit is connected to the output terminal of the amplification stage (110).

14. A medical imaging apparatus, comprising a radiation source (1210) to generate radiation (1211) for the treatment of a living organism to generate an image of at least a portion of the living organism, further comprising the low voltage differential signalling receiver (1231) of claim 13 to process a signal carrying the image.

15. A data processing apparatus, comprising a processor (1310) to process data and a display device (1322) to display information dependent on data processed by the processor, further comprising the low voltage differential signalling receiver (1321) of claim 13 to receive data processed by the processor (1310) and to forward the data to the display device (1322).
